# EUROPEAN PATENT APPLICATION

(11) **EP 4 406 618 A1**
(43) Date of publication of application: **31.07.2024**
(21) Application number: 22872851.5
(22) Date of filing: 16.09.2022
(51) Int. Cl.: A61Q 19/00, B82Y 5/00, B82Y 40/00, A61K 9/127, B01J 13/04, A61K 8/14

(54) **LIPID MEMBRANE ENDOPLASMIC RETICULUM, FABRICATION DEVICE, AND FABRICATION METHOD FOR SAME**

(30) Priority: 21.09.2021 JP 2021152888; 21.09.2021 JP 2021152889; 07.10.2021 JP 2021165726
(71) Applicant: AISIN CORPORATION, Kariya-shi, Aichi 448-8650 (JP)
(72) Inventor: SHIGEMORI, Yasushi, Kariya-shi, Aichi 448-8650 (JP); INOUE, Shinsuke, Kariya-shi, Aichi 448-8650 (JP); HIRANO, Akiyoshi, Kariya-shi, Aichi 448-8650 (JP); TABATA, Yuki, Kariya-shi, Aichi 448-8650 (JP)
(74) Representative: Winter, Brandl - Partnerschaft mbB
(86) International application number: PCT/JP2022/034822
(87) International publication number: WO 2023/048097

(57) **Abstract**

It is a known fact that when fine water having a particle size of 50 nanometers or less enters the horny layer of the skin, the fine water softens the skin and expands gaps in the horny layer. Therefore, when being used for perfumes or cosmetics, skin external preparations, or the like, lipid membrane vesicles containing the fine water are allowed to easily enter the horny layer.

## Description

### TECHNICAL FIELD

The present description discloses a lipid membrane vesicle, and a method and an apparatus for preparing the lipid membrane vesicle.

### BACKGROUND ART

Conventionally, as this type of lipid membrane vesicle, a liposome carrier has been proposed in which h-EGF (Epidermal Growth Factor) is contained as an active ingredient in a liposome formed by forming sphingomyelin into the liposome (see, for example, Patent Literature 1). According to this liposome carrier, the diameter thereof is as very small as 100 nm to 1 µm, and thus the h-EGF can be supplied to skin cells through gaps in the horny layer.

In addition, a liposome composition containing a phospholipid and an amphiphile (arginine hexadecyl phosphate) has also been proposed (see, for example, Patent Literature 2). According to this liposome composition, by lowering the phase transition temperature of the liposome constituent components from around 60°C to around 40°C through appropriate selection of the amphiphile to prepare a more flexible liposome composition, an encapsulated substance can be more easily released and higher transdermal absorbability at the time of use for the skin can be obtained, as compared with the conventional liposome composition.

### CITATIONS LIST

### PATENT LITERATURE

Patent Literature 1: JP 2009-234945 A
Patent Literature 2: JP 2017-171649 A

### SUMMARY OF INVENTION

### TECHNICAL PROBLEMS

Liposomes are generally obtained as heterogeneous mixtures having various particle sizes, and thus there may be many liposomes that cannot penetrate through the horny layer, even when the technique described in Patent Literature 1 is employed. In addition, regarding the liposome composition in Patent Literature 2, it is considered that when an encapsulated substance is released on the surface of the skin due to a decrease in phase transition temperature, the encapsulated substance might not sufficiently permeate into the horny layer. These problems may similarly arise for other lipid membrane vesicles such as extracellular vesicles.

The present invention provides a lipid membrane vesicle having excellent transdermal permeability when the lipid membrane vesicle is used for perfumes or cosmetics, skin external preparations, or the like, and a method and an apparatus for preparing the lipid membrane vesicle.

### SOLUTIONS TO PROBLEMS

In order to achieve the above, the lipid membrane vesicle, and the method and the apparatus for preparing the lipid membrane vesicle, according to the present invention, adopt the following means.

As the gist, a lipid membrane vesicle according to the present invention includes:
fine water having a particle size of 50 nanometers or less; and
a lipid membrane containing the fine water.

It is a known fact that when the fine water having a particle size of 50 nanometers or less enters the horny layer of the skin, the fine water softens the skin and expands gaps in the horny layer. Thus, when the lipid membrane vesicle containing the fine water is used for perfumes or cosmetics, skin external preparations, or the like, the fine water existing on the surface of the lipid membrane vesicle allows the lipid membrane vesicle to easily enter the horny layer. Then, when the fine water enters the horny layer along with the lipid membrane vesicle, the fine water is released from the lipid membrane vesicle and is stored in intercellular lipids (lamellar structure) of the horny layer. Thus, it is possible to enhance a barrier function of the skin and moisturizing power thereof. Here, the lipid membrane may form a lipid bilayer. In this case, the fine water that is retained between the lipid bilayer expands a gap in a horny layer of a skin to promote permeation of the lipid membrane vesicle into the horny layer. In addition, the lipid membrane may be formed in a substantially spherical shape having a diameter of 1 micrometer or less.

As the gist, a method for preparing a lipid membrane vesicle, according to the present invention, includes:
releasing fine water that is uncharged and has a particle size of 50 nanometers or less to a lipid membrane vesicle that has been lyophilized.

According to the method for preparing a lipid membrane vesicle of the present invention, the fine water that is uncharged and has a particle size of 50 nanometers or less is released to the lyophilized lipid membrane vesicle. Thus, it is possible to prepare a lipid membrane vesicle containing a large amount of fine water. Then, by causing the lipid membrane vesicle to contain the fine water, the lipid membrane vesicle can easily enter the horny layer thanks to softening action on the skin brought by the fine water when the lipid membrane vesicle is used for perfumes or cosmetics, skin external preparations, or the like. In addition, the lipid membrane vesicle prepared by causing the lyophilized lipid membrane vesicle to contain the fine water is in a dry state, and thus can exhibit excellent preservation stability by being preserved in this state. Further, by mixing with an aqueous solution selected in accordance with usage, it is possible to easily produce perfumes or cosmetics, or skin external preparations.

As the gist, an apparatus for preparing a lipid membrane vesicle, according to the present invention, includes:
an air passage having an outlet;
an air-blowing unit configured to deliver air into the air passage;
a conductive polymer film provided in the air passage, the conductive polymer film being configured to adsorb, with temperature decrease, moisture in air and to release, with temperature increase, adsorbed moisture as fine water that is uncharged and has a particle size of 50 nanometers or less;
a temperature increasing unit configured to increase a temperature of the conductive polymer film; and
a controller configured to control the air-blowing unit and the temperature increasing unit to discharge the fine water through the outlet toward a lipid membrane vesicle that has been lyophilized.

According to the apparatus for preparing a lipid membrane vesicle of the present invention, by periodically repeating the temperature increase and the temperature decrease of the conductive polymer film, it is possible to uniformly release the fine water that is uncharged and has a particle size of 50 nanometers or less to the lipid membrane vesicle, and it is possible to cause the fine water to be efficiently contained in the lipid membrane vesicle. As a result, it is possible to prepare the lipid membrane vesicle containing the fine water in a short time.

Such an apparatus for preparing a lipid membrane vesicle, according to the present invention, may include a temperature control unit that is installed on a downstream side of the conductive polymer film in the air passage, and that is configured to control a temperature of air flowing through the air passage. With this configuration, it is possible to supply the fine water to the lyophilized lipid membrane vesicle without destroying the lipid membrane vesicle. In this case, a temperature sensor may be provided that is configured to detect a temperature of air containing the fine water that has been discharged through the outlet, the controller may be configured to control the temperature detected by the temperature sensor to be a target temperature, and the target temperature may be a temperature higher than a room internal temperature by 0.5 to 2°C. With this configuration, it is possible to more reliably supply fine water having an appropriate temperature to the lyophilized lipid membrane vesicle.

### BRIEF DESCRIPTION OF DRAWINGS

FIG. 1 is a schematic configuration view of an apparatus for preparing a lipid membrane vesicle including a fine water supply device.
FIG. 2A is a schematic configuration view of a fine water generation cartridge, and FIG. 2B is a schematic configuration view of a fine water generation element.
FIG. 3 is a process chart showing a method for preparing a fine water-containing liposome.
FIG. 4 is a schematic view of a liposome containing fine water.
FIG. 5 is a flowchart showing an example of fine water supply processing.
FIG. 6 is an explanatory view showing a state of the fine water supply device in a moisture absorption mode.

### DESCRIPTION OF EMBODIMENT

A mode for carrying out the present invention will be described with reference to the drawings.

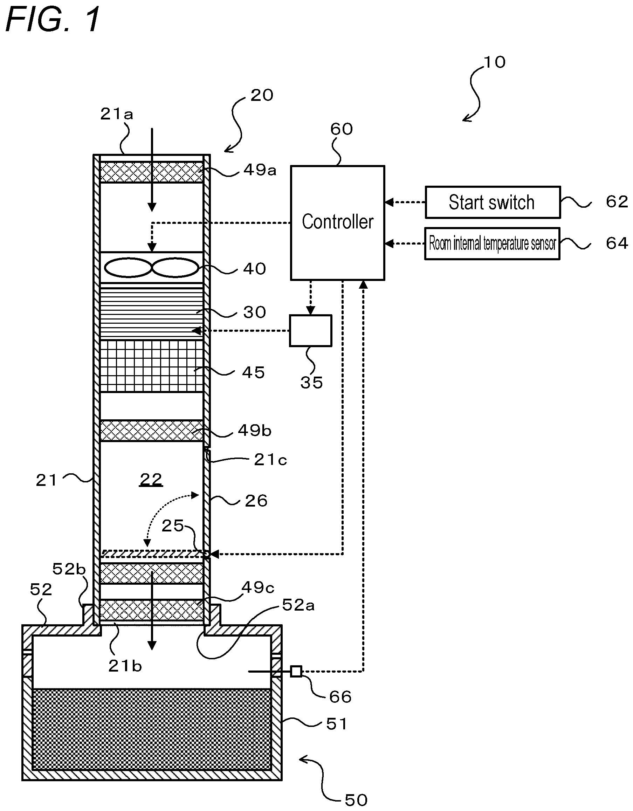

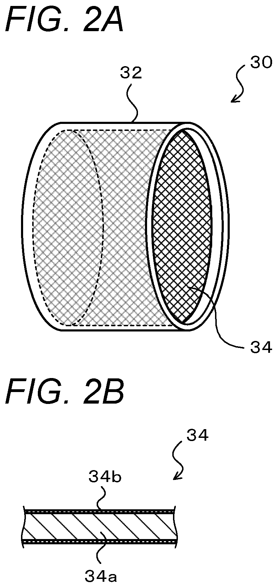

FIG. 1 is a schematic configuration view of an apparatus 10 for preparing a lipid membrane vesicle including a fine water supply device 20. FIG. 2A is a schematic configuration view of a fine water generation cartridge 30, and FIG. 2B is a schematic configuration view of a fine water generation element 34. The preparation apparatus 10 according to the present embodiment includes a container 50, the fine water supply device 20 that supplies fine water toward the container 50, and a controller 60 that controls the entire apparatus. In the present embodiment, lyophilized liposomes are placed in the container 50 as targets to which fine water is to be supplied. The lyophilized liposomes are obtained by lyophilizing a liposome aqueous solution. The liposome aqueous solution can be prepared by dissolving lipids in an organic solvent such as chloroform, distilling off the solvent to form lipid thin membranes, adding an aqueous solution in which substances (active ingredients) to be encapsulated therein are dissolved, and shaking the aqueous solution to hydrate the lipids (Bangham method). Note that the method for preparing the liposome aqueous solution is not limited to the Bangham method, and another preparation method such as a reverse-phase evaporation method may be used. Further, lyophilization can be performed by concentrating the liposome aqueous solution through ultracentrifugation, ultrafiltration, or a combination thereof as necessary, then freezing the solution, and heating and drying the solution under vacuum (vacuum lyophilization). In addition, extracellular vesicles may be placed in the container 50, and fine water may be supplied to the extracellular vesicles. By using naturally derived extracellular vesicles as lipid membrane vesicles, various natural components contained in the extracellular vesicles can be used as active ingredients. Hereinafter, a case in which fine water is supplied to liposomes to prepare fine water-containing liposomes will be described as an example.

The fine water supply device 20 repeatedly switches the state of the fine water generation cartridge 30 between a moisture absorption state and a moisture release state at a predetermined cycle, thereby supplying water particles that are uncharged and have a particle size of 50 nanometers or less in the moisture release state. The fine water supply device 20 includes a duct 21, an energization circuit 35, a fan 40, a temperature control cartridge 45, and filters 49a to 49c, in addition to the fine water generation cartridge 30.

The duct 21 is a tubular member including an air passage 22 that extends vertically and through which air flows. In the duct 21, an upper opening 21a, a lower opening 21b connected to the container 50, and a side opening 21c formed on a side surface of the duct 21 are formed. In addition, the duct 21 is provided with a switching unit 25 for selectively switching between opening and closing of the lower opening 21b and between opening and closing of the side opening 21c.

The switching unit 25 includes a switching plate 26 that operates by driving of a motor (not shown). The switching plate 26 is configured to be able to switch between a first position and a second position. The first position (see the solid lines in FIG. 1) is a position at which the upper opening 21a and the lower opening 21b communicate with each other and the side opening 21c is closed. The second position (see the dotted lines in FIG. 1) is a position at which the upper opening 21a and the side opening 21c communicate with each other and the lower opening 21b is closed. Here, a path connecting the upper opening 21a and the lower opening 21b is referred to as a moisture release path, and a path connecting the upper opening 21a and the side opening 21c is referred to as a moisture absorption path. When the switching plate 26 is located at the first position, the duct 21 enters a state in which the moisture release path is opened and the moisture absorption path is blocked. In contrast, when the switching plate 26 is located at the second position, the duct 21 enters a state in which the moisture release path is blocked and the moisture absorption path is opened.

As shown in FIG. 2A, the fine water generation cartridge 30 includes a cylindrical case 32 having an outer diameter that allows the fine water generation cartridge 30 to be disposed in the air passage 22, and the fine water generation element 34 provided inside the case 32. As shown in FIG. 2B, the fine water generation element 34 includes a base material 34a, and a conductive polymer film 34b formed on a surface of the base material 34a.

The base material 34a is made of a material having electrical conductivity such as a metal material including a stainless-steel-based metal or a copper-based metal, a carbon-based material, or a conductive ceramic material. In the present embodiment, a metal foil made of stainless steel to which aluminum is added is used. Note that the fine water generation element 34 is formed in a corrugated plate shape, a honeycomb shape, a spiral shape, or the like such that air can flow therethrough and the surface area of the base material 34a (conductive polymer film 34b) is made as large as possible. The energization circuit 35 including a power supply and a switch is connected to the base material 34a. When the switch is turned on by the controller 60, the energization circuit 35 is brought into an energized state in which energization to the base material 34a is performed. When the switch is turned off by the controller 60, the energization circuit 35 is brought into a non-energized state in which the energization to the base material 34a is cut off.

The conductive polymer film 34b is made of a polymer compound having electrical conductivity, such as a thiophene-based conductive polymer. In the present embodiment, the conductive polymer film 34b is made of PEDOT/PSS (poly(3,4-ethylenedioxythiophene)/poly(styrene sulfonic acid)) among thiophene-based conductive polymers. PEDOT/PSS is a core-shell structure having a core of PEDOT and a shell of a sulfonic acid group, which is an acidic functional group capable of hydrogen bonding. In addition, in the conductive polymer film 34b, the shells of PEDOT/PSS are aligned to form a stacked structure, and between the shells, a nanochannel, which is a channel having a nanometer size such as 2 nanometers (nm), is formed. In the nanochannel, many sulfonic acid groups exist. Thus, moisture existing on the surface of the conductive polymer film 34b moves, when the amount of the moisture on the surface is larger and the amount of moisture inside the conductive polymer film 34b is smaller, through the sulfonic acid groups in the nanochannel toward the inside, due to a difference in concentration between the surface and the inside. As a result, the conductive polymer film 34b adsorbs the moisture. In addition, in a state where moisture is adsorbed inside, when the amount of moisture on the surface is smaller and the amount of the moisture in the inside is larger, the moisture moves to the surface through the sulfonic acid groups in the nanochannel, due to a difference in concentration between the surface and the inside. As a result, the moisture is released as fine water from the conductive polymer film 34b. In addition, in a state where the temperature of the conductive polymer film 34b increases, more rapid release of moisture (fine water) is promoted as compared with a case in which moisture moves only due to the difference in concentration. In a state where the temperature of the conductive polymer film 34b decreases, more rapid adsorption of moisture is promoted as compared with a case in which moisture moves only due to the difference in concentration. As described above, with a decrease in temperature, the fine water generation cartridge 30 (fine water generation element 34) changes its state to a moisture absorption state in which moisture in the air is adsorbed in the conductive polymer film 34b. With an increase in temperature, the fine water generation cartridge 30 (fine water generation element 34) changes its state to a moisture release state in which the adsorbed moisture is released from the conductive polymer film 34b. Note that the thickness of the conductive polymer film 34b can be appropriately determined in accordance with a necessary adsorption amount (release amount) of fine water. For example, when the conductive polymer film 34b is formed to have a thickness of 1 to 30 micrometers or the like, the conductive polymer film 34b is configured to be able to adsorb moisture sufficient for releasing fine water within a time period of about a few seconds to a few tens of seconds.

Further, the fine water generation cartridge 30 releases, from the conductive polymer film 34b of the fine water generation element 34, uncharged fine water whose water particles have a particle size of 50 nanometers or less, for example, a particle size of about 1 to 2 nanometers. The particle size falls within such a size because of the following conceivable reason. That is, by reason of the size of the nanochannel being 2 nanometers or less, a phenomenon is considered to occur in which moisture is impulsively released from the nanochannel because of an improvement in mobility of water and an increase in pressure in the nanochannel caused by an increase in temperature of the conductive polymer film. In addition, even if water particles aggregate after impulsive release, the particle size is distributed within a range of 50 nanometers or less. Detailed description of such fine water generation in the fine water generation cartridge 30 (conductive polymer film 34b) is described in WO 2020/054100 A, JP 2019-018195 A, and the like of the present applicant, and thus further detailed description is omitted.

When the fan 40 is rotationally driven in a first rotational direction, the fan 40 causes air flow from the upper side toward the lower side of the duct 21. Thus, by moving the switching plate 26 to the first position to open the moisture release path and block the moisture absorption path (see the solid lines in FIG. 1), air can be sucked into the air passage 22 from the upper opening 21a of the duct 21 through the filter 49a, and the sucked air can be sent to the container 50, as air containing fine water, through the fine water generation cartridge 30 and the filters 49b, 49c (moisture release mode). In addition, when the fan 40 is rotationally driven in a second rotational direction opposite to the first rotational direction, the fan 40 causes air flow from the lower side toward the upper side of the duct 21. Thus, by moving the switching plate 26 to the second position to block the moisture release path and open the moisture absorption path (see the dotted lines in FIG. 1), air can be sucked into the air passage 22 from the side opening 21c of the duct 21 through the filter 49b, and the sucked air can be sent to the outside through the upper opening 21a while moisture in the sucked air is absorbed by the fine water generation cartridge 30 (moisture absorption mode).

The fan 40 is rotationally driven by a motor (not shown), and is controlled by the controller 60 with PWM (Pulse Width Modulation) control. Note that the fan 40 may be a propeller fan, a sirocco fan, or the like.

The temperature control cartridge 45 is made of a metal material, while the temperature control cartridge 45 has, for example, a corrugated plate shape, a honeycomb shape, a spiral shape, or the like, to allow the temperature control cartridge 45 to have a large thermal capacity and high heat exchange efficiency. The temperature control cartridge 45 is disposed below the fine water generation cartridge 30. Thus, by the fan 40 being rotationally driven in the first rotational direction, air having passed through the fine water generation cartridge 30 and flowing to the temperature control cartridge 45 is cooled by heat exchange when the air passes through the temperature control cartridge 45, in the moisture release mode.

The container 50 includes a container body 51 whose upper portion is opened to accept a target of supply, and a top lid 52 attached to the upper portion of the container body 51. The top lid 52 has an upper surface, on which a top lid opening 52a is formed to be opened substantially at the center with substantially the same diameter as the lower opening 21b of the duct 21, and on which a support portion 52b is also formed to be erected upward from the edge of the top lid opening 52a, supporting the lower end portion of the duct 21. Thus, as shown in the drawing, the duct 21 is supported by the support portion 52b, whereby the fine water supply device 20 can be placed on the upper portion of the container 50, as a result of which air containing fine water can be fed from the fine water supply device 20 into the container 50 to fill the container 50. Note that, although not shown, in the top lid 52, an exhaust valve is formed, which exhausts air to the outside when internal pressure in the container 50 increases.

The controller 60 is configured as a microprocessor mainly including a CPU, and includes a ROM, a RAM, and input-output ports in addition to the CPU. The controller 60 receives input, via the input ports, of various signals including an operation signal from a start switch 62 for starting the operation of the preparation apparatus 10, a temperature signal from a room internal temperature sensor 64, and a temperature signal from a temperature sensor 66 placed inside the container 50. In addition, the controller 60 outputs, via the output ports, various signals including a driving signal to the motor that rotationally drives the fan 40, a driving signal to the switch of the energization circuit 35, and a driving signal to the motor of the switching unit 25.

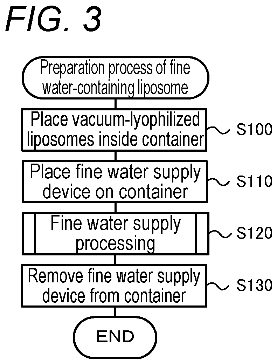

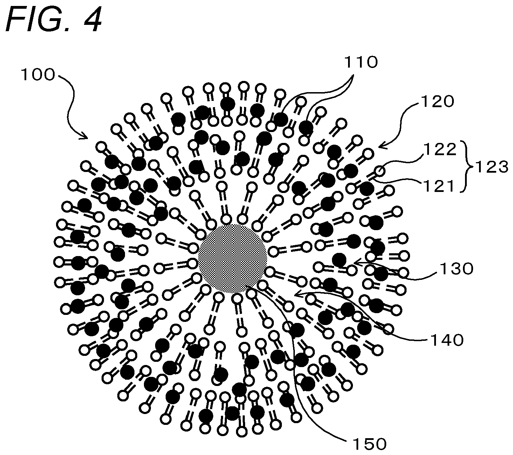

Next, a method for preparing a liposome (fine water-containing liposome) will be described by using the preparation apparatus 10 configured as described above. FIG. 3 is a process chart showing the method for preparing the fine water-containing liposome. An operator first places the lyophilized liposomes described above inside the container body 51, and attaches the top lid 52 to the container body 51 (S100). Next, the operator places the fine water supply device 20 (duct 21) on the top lid 52 of the container 50 (5110). Then, the operator operates the start switch 62 to cause the fine water supply device 20 to execute fine water supply processing (S120). Details of the fine water supply processing will be described later. When the fine water supply processing is completed, the operator removes the fine water supply device 20 from the container 50 (S130), and fine water-containing liposomes are prepared. As shown in FIG. 4, a fine water-containing liposome 100 is a liposome prepared by causing a lyophilized liposome 100 to contain fine water 110 having a particle size of 50 nanometers or less. The fine water-containing liposome 100 has substantially spherical lipid membranes 120, 130, 140, which are layered together. The lipid membrane 120 forms a lipid bilayer in which phospholipids 123 each having a hydrophobic portion 121 and a hydrophilic portion 122 are arranged such that each hydrophobic portion 121 is oriented to the inner side and each hydrophilic portion 122 is oriented to the outer side. The lipid membranes 130, 140 are formed similarly to the lipid membrane 120. The fine water 110 stays between (inside) the lipid bilayer. An active ingredient 150 such as a drug may be encapsulated in the central portion of the fine water-containing liposome 100. The fine water-containing liposome 100 has a diameter of 60 nm to 1 µm, and can enter the inside of the horny layer through gaps (each about 180 nm) in the horny layer. After the fine water enters the horny layer of the skin and softens the skin to expand the gaps in the horny layer, the fine water-containing liposome 100 can more easily enter the horny layer. The fine water-containing liposome 100 is in a dry state, and thus can exhibit excellent preservation stability by being preserved in this state. Further, by mixing various aqueous solutions in accordance with usage, it is possible to easily produce perfumes or cosmetics, or skin external preparations.

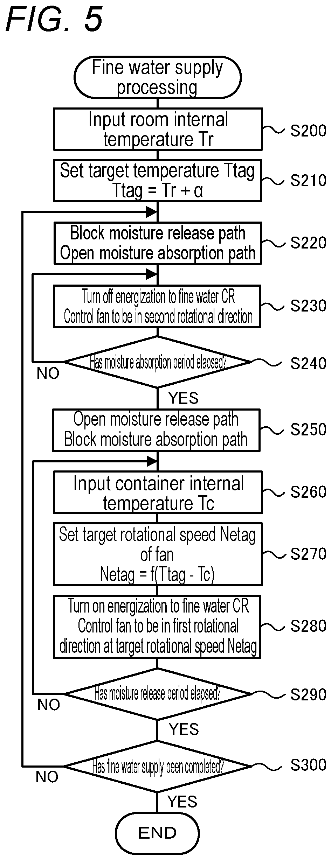

FIG. 5 is a flowchart showing an example of the fine water supply processing. In the fine water supply processing, first, the controller 60 inputs a room internal temperature Tr provided from the room internal temperature sensor 64 (S200), and sets a temperature obtained by adding a predetermined temperature α (for example, 0.5°C to 2°C) to the input room internal temperature Tr, as a target temperature Ttag inside the container 50 (S210).

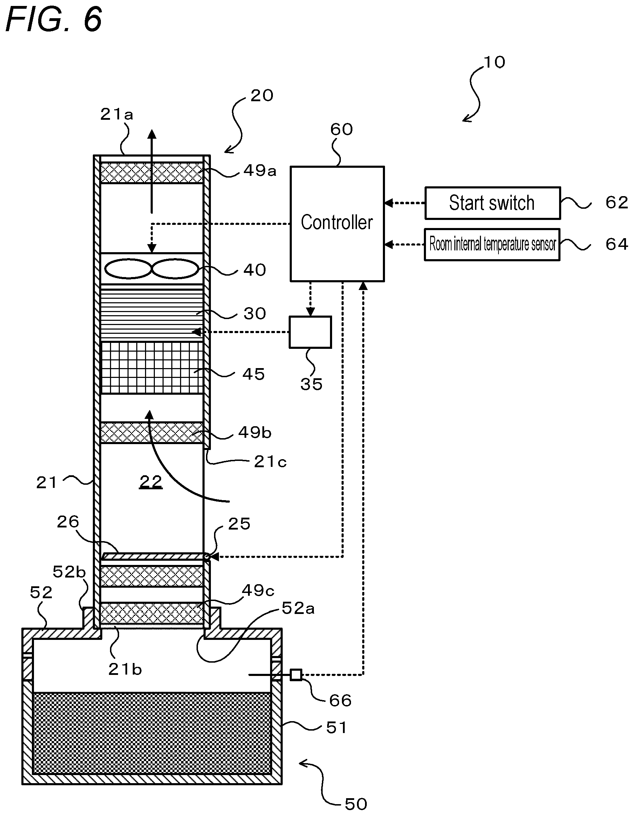

Next, the controller 60 drives the switching unit 25 (motor) such that the switching plate 26 is located at the second position (S220). As a result, as shown in FIG. 6, the duct 21 is brought into a state in which the moisture release path is blocked and the moisture absorption path is opened. Then, while the controller 60 turns off the energization to the fine water generation cartridge 30, the controller 60 performs moisture absorption control in which the motor is controlled such that the fan 40 is rotationally driven in the second rotational direction at a predetermined rotational speed determined in advance to suck air from the side opening 21c of the duct 21 (the moisture absorption path), to cause the sucked air to pass through the fine water generation cartridge 30, and then to discharge the sucked air through the upper opening 21a (see the solid arrows in FIG. 6) (S230). When the controller 60 performs the moisture absorption control, the controller 60 determines whether a predetermined moisture absorption period has elapsed (S240). In a case where the controller 60 determines that the moisture absorption period has not elapsed, the controller 60 returns to S230, and continues the moisture absorption control.

In contrast, in a case where the controller 60 determines that the moisture absorption period has elapsed, the controller 60 drives the switching unit 25 (motor) such that the switching plate 26 is located at the first position (S250). As a result, the duct 21 is brought into a state in which the moisture release path is opened and the moisture absorption path is blocked. Next, the controller 60 inputs a container internal temperature Tc provided from the temperature sensor 66 (S260), and sets a target rotational speed Netag of the fan 40 through feedback calculation based on deviation between the input container internal temperature Tc and the target temperature Ttag set in S210 (S270). This processing is calculation processing in feedback control for matching the container internal temperature Tc with the target temperature Ttag, and can be performed by using, for example, proportional-integral calculation or proportional-integral-differential calculation. Then, the controller 60 turns on the energization to the fine water generation cartridge 30, and performs moisture release control in which the motor is controlled such that the fan 40 is rotationally driven in the second rotational direction at the target rotational speed Netag set in S260 to suck air from the upper opening 21a of the duct 21 (the moisture release path), to cause the sucked air to pass through the fine water generation cartridge 30, and then to discharge the sucked air through the lower opening 21b (see the solid arrows in FIG. 1) (S280). When the controller 60 performs the moisture release control, the controller 60 determines whether a predetermined moisture release period has elapsed (S290). In a case where the controller 60 determines that the moisture release period has not elapsed, the controller 60 returns to S260, and continuously performs the moisture release control such that the container internal temperature Tc matches the target temperature Ttag.

In a case where the controller 60 determines that the moisture release period has elapsed, the controller 60 determines whether the supply of fine water has been completed (S300). In a case where the controller 60 determines that the supply of the fine water has not been completed, the controller 60 returns to S220, and performs the moisture absorption control. As described above, by causing the controller 60 to repeatedly perform the moisture absorption control and the moisture release control, fine water can be supplied to the container 50. The moisture absorption time period and the moisture release time period can be appropriately set in accordance with moisture absorption capacity (moisture release capacity) of the fine water generation cartridge 30, the size of the container 50, or the like. For example, the moisture absorption time period can be set to a time period about twice as much as the moisture release time period. As an example of this, the moisture absorption time period can be set to 1 minute or 2 minutes in a case where the moisture release time period is set to 30 seconds or 1 minute.

In addition, in S300, the determination as to whether the supply of the fine water has been completed can be made by determining whether a predetermined time period has elapsed since the start of the fine water supply processing, or by determining whether the number of times of repetition of the moisture absorption control and the moisture release control has reached a predetermined number of times. Here, the execution time period of the fine water supply processing can be appropriately determined in accordance with the amount of lyophilized liposomes that are targets to which fine water is to be supplied. However, the execution time period can be, for example, two hours or more, or three hours or more, in order to cause the lyophilized liposomes to contain a sufficient amount of fine water. When the controller 60 determines, in S300, that the supply of the fine water has been completed, the controller 60 ends the fine water supply processing.

The apparatus 10 for preparing a lipid membrane vesicle according to the present embodiment described above includes the fine water supply device 20 including the fine water generation cartridge 30 (conductive polymer film 24b). Thus, it is possible to uniformly release fine water, which is uncharged and has a particle size of 50 nanometers or less, to lyophilized liposomes by repeatedly changing the state of the conductive polymer film 24b to the moisture absorption state and the moisture release state. Therefore, it is possible to cause the fine water to be efficiently contained in the liposomes, and it is possible to prepare liposomes containing the fine water in a short time. The lyophilized liposomes containing the fine water are in a dry state, and thus can exhibit excellent preservation stability. In addition, by mixing an aqueous solution containing an active ingredient, it is possible to easily produce perfumes or cosmetics, or skin external preparations from the lyophilized liposomes.

In addition, in the preparation apparatus 10 according to the present embodiment, the target temperature Ttag inside the container 50 is set to a temperature higher than the room internal temperature Tr by about 0.5°C to 2°C, and control is performed such that the container internal temperature Tc, provided from the temperature sensor 66 placed inside the container 50, matches with the target temperature Ttag. Thus, it is possible to supply fine water to liposomes while reducing destruction of the liposomes due to temperature.

Here, when the liposomes containing fine water are used for the skin as one of perfumes or cosmetics, or a skin external preparation, the fine water contained in the vicinity of the surface of the liposomes can easily permeate through the horny layer after the liposomes containing the fine water are applied to the skin as one of perfumes or cosmetics, or a skin external preparation, because the fine water has a particle size of 50 nanometers or less. In addition, it is a known fact that when fine water permeates through the horny layer, the fine water softens the skin and expands gaps in the horny layer (Nishimura N, Inoue S, Yokoyama K, Iwase S. "Effect of spraying of fine water particles on facial skin moisture and viscoelasticity in adult women," Skin Res Technol. 2019; 25: 294-298.). Thus, it is possible to promote permeation of the liposomes into the horny layer.

Then, when the liposomes enter the horny layer, the liposomes stay in the horny layer, and the encapsulated active ingredients and fine water are released with the lapse of time. Thus, the effect of the active ingredients can be exerted. Further, the fine water is stored in intercellular lipids (lamellar structure) of the horny layer, and can enhance a barrier function of the skin and moisturizing power thereof.

Note that, in the embodiment described above, the case has been described, as an example, in which the fine water-containing liposomes are prepared by supplying the fine water to the lyophilized liposomes. However, extracellular vesicles also have lipid membranes similar to those of the liposomes, and thus it is considered that the fine water can be contained inside the extracellular vesicles similarly to the liposomes by supplying the fine water to the extracellular vesicles that have been lyophilized, and that an effect similar to that of the fine water-containing liposomes can be exhibited.

In the embodiment described above, the temperature control cartridge 45 is provided between the fine water generation cartridge 30 and the lower opening 21b in the duct 21. Then, the temperature control is performed in the container 50 by controlling, through the fan 40, the amount of air passing through the temperature control cartridge 45. However, the temperature control may be performed by controlling an amount of energization to the fine water generation cartridge 30 (base material 34a) through the energization circuit 35 to control the temperature of the fine water generation cartridge 30 (conductive polymer film 34b). Alternatively, the temperature control may be performed by a temperature controller such as a Peltier device, after providing the temperature controller between the fine water generation cartridge 30 and the lower opening 21b in the duct 21. Alternatively, the temperature control may be performed on the basis of a combination of any two or more of the aboves. Alternatively, an option may be adopted in which such temperature control is not performed.

In the embodiment described above, the fine water is supplied to the container 50 by changing the state of the single fine water generation cartridge 30 to the moisture absorption state and the moisture release state at a predetermined cycle. However, a plurality of fine water generation cartridges 30 may be arranged in parallel with respect to the container 50, and fine water may be continuously supplied to the container 50, by repeating the moisture absorption state and the moisture release state while causing phases to be different from each other such that when one of the plurality of fine water generation cartridges 30 enters the moisture absorption state, another fine water generation cartridge 30 enters the moisture release state.

In the embodiment described above, the fine water supply device 30 alternately repeats the moisture absorption period during which the moisture absorption control is performed and the moisture release period during which the moisture release control is performed. However, an idle period during which neither the moisture absorption control nor the moisture release control is performed may be provided between the moisture absorption period and the moisture release period.

The correspondence relationship between the main elements of the embodiment and the main elements of the invention described in the section of SOLUTIONS TO PROBLEMS will be described. In the embodiment, the lower opening 21b corresponds to the "outlet", the air passage 22 corresponds to the "air passage", the fan 40 corresponds to the "air-blowing unit", the conductive polymer film 34b of the fine water generation cartridge 30 corresponds to the "conductive polymer film", the energization circuit 35 corresponds to the "temperature increasing unit", and the controller 60 corresponds to the "controller". In addition, the temperature sensor 66 corresponds to the "temperature sensor".

Note that the correspondence relationship between the main elements of the embodiment and the main elements of the invention described in the section of SOLUTIONS TO PROBLEMS does not limit the elements of the invention described in the section of SOLUTIONS TO PROBLEMS, because the embodiment is an example for specifically describing a mode for carrying out the invention described in the section of SOLUTIONS TO PROBLEMS. That is, the invention described in the section of SOLUTIONS TO PROBLEMS should be construed on the basis of the description in the section, and the embodiment is merely a specific example of the invention described in the section of SOLUTIONS TO PROBLEMS.

While the mode for carrying out the present invention has been described with the embodiment, the present invention is not limited to such an embodiment at all, and it goes without saying that the present invention can be carried out in various modes without departing from the gist of the present invention.

### INDUSTRIAL APPLICABILITY

The present invention can be used for, for example, industries for producing perfumes or cosmetics, skin external preparations, or the like.

### REFERENCE SIGNS LIST

10: Apparatus for preparing lipid membrane vesicle, 20: Fine water supply device, 21: Duct, 21a: Upper opening, 21b: Lower opening, 21c: Side opening, 22: Air passage, 24b: Conductive polymer film, 25: Switching unit, 26: Switching plate, 30: Fine water generation cartridge, 32: Case, 34: Fine water generation element, 34a: Base material, 34b: Conductive polymer film, 35: Energization circuit, 40: Fan, 45: Temperature control cartridge, 49a, 49b, 49c: Filter, 50: Container, 51: Container body, 52: Top lid, 52a: Top lid opening, 52b: Support portion, 60: Controller, 62: Start switch, 64: Room internal temperature sensor, 66: Temperature sensor, 100: Fine water-containing liposome, 110: Fine water, 120, 130, 140: Lipid membrane, 121: Hydrophobic portion, 122: Hydrophilic portion, and, 123: Phospholipid

## Claims

1. A lipid membrane vesicle, comprising:
fine water having a particle size of 50 nanometers or less; and
a lipid membrane containing the fine water.

2. The lipid membrane vesicle according to claim 1, wherein the lipid membrane forms a lipid bilayer.

3. The lipid membrane vesicle according to claim 2, wherein the fine water that is retained between the lipid bilayer expands a gap in a horny layer of a skin to promote permeation of the lipid membrane vesicle into the horny layer.

4. The lipid membrane vesicle according to claim 1, wherein the lipid membrane is formed in a substantially spherical shape having a diameter of 1 micrometer or less.

5. A method for preparing a lipid membrane vesicle, the method comprising releasing fine water that is uncharged and has a particle size of 50 nanometers or less to a lipid membrane vesicle that has been lyophilized.

6. An apparatus for preparing a lipid membrane vesicle, the apparatus comprising:
an air passage having an outlet;
an air-blowing unit configured to deliver air into the air passage;
a conductive polymer film provided in the air passage, the conductive polymer film being configured to adsorb, with temperature decrease, moisture in air and to release, with temperature increase, adsorbed moisture as fine water that is uncharged and has a particle size of 50 nanometers or less;
a temperature increasing unit configured to increase a temperature of the conductive polymer film; and
a controller configured to control the air-blowing unit and the temperature increasing unit to discharge the fine water through the outlet toward a lipid membrane vesicle that has been lyophilized.

7. The apparatus for preparing a lipid membrane vesicle according to claim 6, the apparatus further comprising a temperature control unit installed on a downstream side of the conductive polymer film in the air passage, the temperature control unit being configured to control a temperature of air flowing through the air passage.

8. The apparatus for preparing a lipid membrane vesicle according to claim 7, the apparatus further comprising a temperature sensor configured to detect a temperature of air containing the fine water that has been discharged through the outlet,
wherein
the controller is configured to control the temperature detected by the temperature sensor to be a target temperature, and
the target temperature is a temperature higher than a room internal temperature by 0.5 to 2°C.
